# EUROPÄISCHE PATENTSCHRIFT

(11) **EP 1 091 809 B1**
(45) Veröffentlichungstag und Bekanntmachung des Hinweises auf die Patenterteilung: **10.09.2003**
(21) Anmeldenummer: 99929267.5
(22) Anmeldetag: 18.06.1999
(51) Int. Cl.: B05B 11/00

(54) **APPLIKATIONS-VORRICHTUNG FÜR KEIMFREIE FLUIDE**
APPLICATION DEVICE FOR GERM-FREE FLUIDS
DISPOSITIF D'APPLICATION POUR FLUIDES STERILES

(30) Priorität: 24.06.1998 DE 29811242 U
(43) Veröffentlichungstag der Anmeldung: 18.04.2001
(73) Patentinhaber: MERCK PATENT GmbH, 64293 Darmstadt (DE)
(72) Erfinder: OSWALD, Klaus, D-64560 Riedstadt (DE)
(86) Internationale Anmeldenummer: EP9904226
(87) Internationale Veröffentlichungsnummer: WO99067025

(56) Entgegenhaltungen:
- DE-A- 2 709 796
- FR-A- 2 643 338
- US-A- 4 117 957
- US-A- 5 692 649

## Beschreibung

Die Erfindung betrifft eine Applikations-Vorrichtung für keimfreie Fluide, insbesondere für pharmazeutisch wirksame Flüssigkeiten, bestehend aus einem das keimfrei abgefüllte Fluid ohne Luftraum aufnehmenden Behälter, in dessen abgabeseitiger Begrenzungswand eine luftausgleichsfrei arbeitende, als Hubkolbenpumpe ausgebildete Dosierpumpe mit einem eine Einlaßöffnung zum Behälter verschließenden Einlaßventil und einem abgabeseitig eine zu einer Abgabeöffnung geführten Verbindung verschließenden Auslaßventil, wobei der Kolben der Dosierpumpe federnd in eine Hub-Endstellung vorgespannt ist, aus welcher er durch ein relativ zum Behälter verschiebliches, mit der Abgabeöffnung versehenes. Betätigungselement durch den Benutzer von Hand in die andere Hub-Endstellung verschieblich ist, wobei durch den bei der Hubbetätigung in dem in der Pumpe eingeschlossenen Fluid entstehenden Druck das Einlaßventil geschlossen und das Auslaßventil geöffnet wird.

Eine derartige Vorrichtung ist aus US 4 117 957 bekannt.

Bis heute werden dosiert zu applizierende pharmazeutisch wirksame Flüssigkeiten, beispielsweise schleimhautabschwellende und entzündungshemmende, durch Einsprühen in die Nase eines Patienten zu applizierende Flüssigkeiten in relativ einfach aufgebauten Sprühbehältern aus elastisch zusammendrückbarem Kunststoff abgefüllt. Beim Applizieren der Flüssigkeit wird ein langgestreckter Sprüh- oder Spritzansatz des Behälters in das jeweilige Nasenloch eingeführt und der Behälter zusammengedrückt, wobei die im Behälter abgefüllte Flüssigkeit über ein Steigrohr zu einer Düsenöffnung im Ansatz verdrängt und dann in Form möglichst feiner Tröpfchen ausgesprüht wird. Bei der anschließenden Rückverformung des Behälters wird zur Vermeidung eines Vakuums im Behälter Umgebungsluft ins Behälterinnere zurückgesaugt, wobei zwangsläufig in der Luft enthaltene Keime ins Behälterinnere gelangen und die noch im Behälter befindliche Flüssigkeit verkeimen können. Es ist deshalb üblich, den Flüssigkeiten Konservierungsmittel zuzusetzen, welche eine Vermehrung von Keimen verhindern. Dabei ist nicht auszuschließen, daß die Konservierungsmittel die pharmazeutische Wirksamkeit der zur applizierenden Flüssigkeit beeinträchtigen. In Einzelfäilen können auch allergische Reaktionen der Benutzer infolge des Konservierungsmittels nicht ausgeschlossen werden. In der DE 39 00 267 A1 ist ein Spender zur portionierten Ausgabe von viskosen Substanzen offenbart; bei einer derartigen Aufgabenstellung sind Probleme unerheblich, wie sie bei einer Applikations-Vorrichtung für keimfreie Fluide, insbesondere bei Vorrichtungen zum Versprühen auftreten.

Es besteht daher die Forderung, Auftrage-Vorrichtungen für die dosierte Abgabe von keimfrei, jedoch ohne Konservierungsmittel abgefüllte Flüssigkeiten zu schaffen. Bei einer bekannten Vorrichtung der eingangs erwähnten Art (DE 40 27 320 C2) wird dies dadurch erreicht, daß im Einlaßventil und/oder dessen Zulauf und/oder Ablauf oligodynamisch wirksame Substanz, d.h. ein antimikrobiell wirksamer Stoff vorgesehen wird, welcher aufgrund seiner keimabtötenden Wirkung sicherstellt, daß beim Gebrauch der Vorrichtung bis in den Bereich des Einlaßventils der Dosierpumpe eingedrungene Keime abgetötet werden und so die im eigentlichen Behälter befindliche Flüssigkeit nicht verkeimt werden kann. In der bekannten Abgabevorrichtung kann die zu applizierende Flüssigkeit also bei keimfreier Abfüllung auch ohne Zusatz von Konservierungsstoffen für eine gewisse Zeitdauer, d.h. bis zum Verbrauch, aufbewahrt werden, ohne daß eine unzulässige Verkeimung beobachtet wird. Durch den erforderlichen Einsatz der oligodynamisch wirksamen Substanz, beispielsweise einer Silberschicht oder einem Silberdepot aus schwer löslichem Silbersalz wird die Herstellung der bekannten Abgabevorrichtung aufwendig und somit teuer, zumal eine Wiederverwendung ja nicht möglich ist.

Der Erfindung liegt demgegenüber die Aufgabe zugrunde, eine Applikations-Vorrichtung für konservierungsstofffreie keimfrei abzufüllende Flüssigkeiten zu schaffen, welche preiswerter herstellbar ist und eine Verkeimung der zu applizierenden Flüssigkeit während der Gebrauchsdauer mit Sicherheit vermeidet, wobei die Vorrichtung insbesondere für den Sprühauftrag geeignet ausgebildet sein soll. Als "keimfrei" im Sinne der Erfindung werden dabei auch solche Lösungen verstanden, bei denen in der zu applizierenden Flüssigkeit während des bestimmungsgemäßen Gebrauchs eine nur geringe, entsprechend dem Verwendungszweck tolerable bzw. gesetzlich zulässige Verkeimung auftritt.

Ausgehend von einer Applikations-Vorrichtung der eingangs erwähnten Art wird diese Aufgabe erfindungsgemäß dadurch gelöst, daß die den gefüllten Behälter auf der der Einlaßöffnung gegenüberliegenden Seite verschließende Wand als im Behälter verschieblich geführter und entlang seines Umfangs an der Innenwandung des Behälters abdichtender Kolben ausgebildet ist, und daß der Ventilkörper des Auslaßventils mit einem sich kegelstumpfförmig erweiternden Düsen-Verschlußkörper verbunden ist, dessen Kegelstumpf-Umfangsfläche in der Ventil-Schließstellung an einem sich komplementär kegelstumpfförmig erweiternden Bereich der Abgabeöffnung abdichtend anliegt. Die Abgabeöffnung wird in ihrem äußeren kegelstumpfförmigen Bereich also im Zusammenwirken mit dem Düsen-Verschlußkörper als Sprühdüse ausgebildet, wobei der Verschlußkörper nur während des Sprühvorgangs geöffnet ist und bei nachlassendem Druck in der Dosierpumpe sofort schließt, so daß ein Zurücksaugen von bereits ausgetretener Flüssigkeit oder gar der Eintritt von Umgebungsluft in die Dosierpumpe mit Sicherheit vermieden ist. Damit ist auch der Übertritt von Keimen aus der Umgebung in die zu applizierende Flüssigkeit ausgeschlossen. Ein Luftraum innerhalb des die Flüssigkeit aufnehmenden Behälters kann nicht entstehen, weil der Innenraum des Behälters durch den infolge des atmosphärischen Drucks bei Verringerung der Behälter-Füllmenge nachgeschobenen Kolben entsprechend der entnommenen Flüssigkeitsmenge verkleinert wird.

Der an der Innenwandung des Behälters abdichtende Kolben weist vorzugsweise entlang seiner behälterinnenseitig an der Innenwand des Behälters anliegenden Randes eine umlaufende Lippendichtung auf, welche den Austritt von Flüssigkeit verhindert. Außerdem weist der Kolben zweckmäßig auch entlang seines behälteraußenseitig an der Innenwand des Behälters anliegenden Rands eine umlaufende Lippendichtung auf, welche den Eintritt von Luft aus der Umgebungsatmosphäre verhindert. Dafür ist es zweckmäßig, wenn die Lippendichtung(en) unter elastischer Vorspannung an die Innenwandung des Behälters angedrückt ist bzw. sind.

Der rechtwinklig zur Verschiebungsrichtung des Kolbens verlaufende lichte innere Querschnitt des Behälters wird dann mit Vorteil in seinem den Kolben verschieblich aufnehmenden Bereich komplementär geometrisch zur äußeren Begrenzung des Kolbens begrenzt, wobei der Behälter und Kolben kreisförmig, elliptisch oder auch polygonal mit abgerundeten Polygonecken versehene Begrenzungen haben können.

An der ins Behälterinnere weisenden Kegelstumpf-Stirnfläche des mit dem Ventilkörper des Auslaßventils des Düsen-Verschlußkörpers ist in vorteilhafter Weiterbildung der Erfindung ein langgestreckter Schaft angesetzt, der mit dem kolbenartig ausgebildeten, in einer zylindrischen Sitzfläche verschieblich geführten Ventilkörper des Auslaßventils verbunden ist, wobei der Ventilkörper durch eine Feder in eine Endstellung vorgespannt ist, in welcher die Kegelstumpf-Mantelfläche des Düsen-Verschlußkörpers am komplementär kegelstumpfförmigen Bereich der Abgabeöffnung anliegt und der Ventilkörper seinerseits eine oder mehrere Durchlaßöffnung(en) in der Wandung der Sitzfläche verschließt, wobei die Durchlaßöffnung(en) über wenigstens einen anschließenden Kanal mit der Abgabeöffnung verbunden ist bzw. sind. Der Ventilkörper und der Düsen-Verschlußkörper sind also zwangsläufig so verbunden, daß sie beim Applikationsvorgang durch den in der Dosierpumpe der Flüssigkeit entstehenden Druck gleichzeitig geöffnet und geschlossen werden.

Der Kolben der Dosierpumpe ist zweckmäßig - in an sich bekannter Weise - durch eine Schraubenfeder in die Hub-Endstellung vorgespannt, wobei die Schraubenfeder im Innern eines den Zylinder der Dosierpumpe bildenden hohlen Ansatzes der Begrenzungswand angeordnet sein kann.

In vorteilhafter Abwandlung kann die Schraubenfeder demgegenüber auch einen den von der Begrenzungskante des Behälters vortretenden Ansatz im wesentlichen konzentrisch umgeben und ist dann auf der Außenseite der Begrenzungswand und den von einer Überkappe gebildeten Betätigungselement andererseits abgestützt. Im letztgenannten Fall ist die Ausgestaltung dann bevorzugt so getroffen, daß der eine Durchgangsöffnung aufweisende langgestreckte Kolben der Dosierpumpe an seinem behälterseitigen Endbereich in dem im wesentlichen hohlzylindrischen Ansatz gehalten und an seinem ins Behälterinnere weisenden Ende mit dem Einlaßventil versehen ist, während sein behälterabgewandtes Ende verschieblich ins Innere des in der Sprühdüse angeordneten Zylinders eingreift, in dessen behälterabgewandten Endbereich der Ventilkörper des Aulaßventils angeordnet ist.

Die Erfindung ist in der folgenden Beschreibung zweier Ausführungsbeispiele in Verbindung mit der Zeichnung näher erläutert, und zwar zeigt:
- Fig. 1: einen Längsmittelschnitt durch ein erstes Ausführungsbeispiel einer erfindungsgemäßen Applikations-Vorrichtung, und
- Fig. 2: einen Längsmittelschnitt durch ein zweites Ausführungsbeispiel einer erfindungsgemäßen Applikations-Vorrichtung.

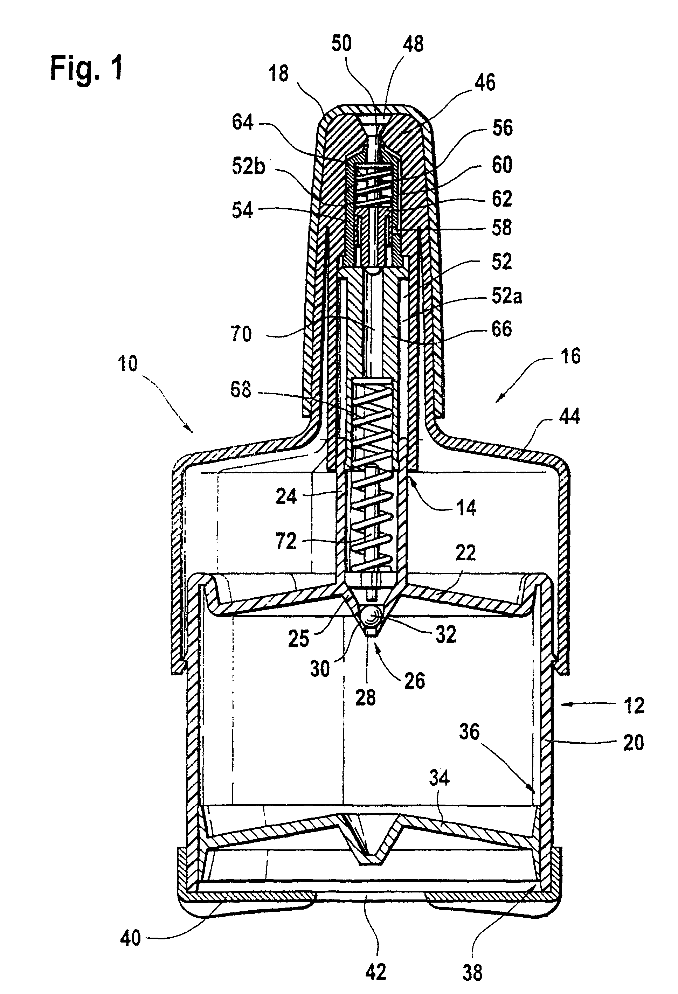

Die in Fig. 1 gezeigte in ihrer Gesamtheit mit 10 bezeichnete Applikations-Vorrichtung weist insgesamt drei Funktions-Baugruppen auf, nämlich den die zur applizierende Flüssigkeit aufnehmenden Behälter 12, die Dosierpumpe 14 und die gleichzeitig zur Betätigung der Dosierpumpe 14 dienende Sprühdüse 16 auf, zu denen dann noch eine bei Nichtgebrauch auf der Sprühdüse aufschiebbare Schutzkappe 18 hinzutritt.

Der Behälter 12 weist im dargestellten Fall einen mittleren zylindrischen Abschnitt 20 auf, der an seinem in der Zeichnung oben liegenden Ende durch eine integral angesetzte Deck- oder Begrenzungswand 22 verschlossen ist, von welcher mittig ein langgestreckter, an seinem oberen Ende offener zylindrischer Ansatz 24 integral vortritt, welcher Teil des Gehäuses, nämlich der Zylinder der nachstehend noch im einzelnen beschriebenen Dosierpumpe 14 ist. Am unteren Ende des Ansatzes 24 ist innerhalb eines in den Behälter ragenden kegelstumpfförmigen Vorsprungs 25 ein Einlaßventil 26 gebildet, welches aus einem im Anschluß an eine Einlaßöffnung 28 in der ansatzabgewandten Begrenzungsfläche des Vorsprungs 24 ausgebildeten Ventil-Sitzfläche 30 gebildet wird, die mit einer Ventil-Kugel 32 zusammenwirkt.

An der der Begrenzungswand 22 gegenüberliegenden Seite mündet der Behälter 12 offen. In die offene Mündung ist ein an der Innenseite der Behälterwandung abdichtender Kolben 34 in Richtung der Behälter-Mittelachse verschieblich eingesetzt. Die umlaufenden Ränder der an der Behälter-Innenwand anliegenden Umfangswandung des Kolbens sind als einerseits ins Behälterinnere und andererseits aus dem Behälterinnern herausweisende Lippendichtungen 36 und 38 ausgebildet, die unter leichter radialer Vorspannung an der Behälter-Innenwandung anliegen. Die offene Mündung des Behälters wird dann noch durch eine den Randbereich übergreifende Bodenkappe 40 abgeschlossen, welche den Behälter 12 in seiner Form stabilisiert und eine mittige Öffnung 42 aufweist, über welche der atmosphärische Druck auf die zugewandte Außenseite des Kolbens 34 einwirkt, wobei anstelle dieser mittigen Öffnung 42 auch mehrere oder eine nicht mittige Öffnung vorgesehen sein können.

Die Sprühdüse 16 ist in ihrem behälterseitigen Endbereich als die zylindrische Behälterwandung 20 übergreifende und auf dieser Behälterwandung verschiebliche Überkappe 44 ausgebildet, die an ihrer behälterabgewandten Seite in einen mittig vorspringenden langgestreckten hohlen Düsenkörper 46 übergeht, welcher zum Applizieren einer im Behälter 12 abgefüllten Flüssigkeit in eine Körperöffnung des Benutzers - z.B. eine Nasenöffnung oder den Mund - einführbar ist. Im freien Ende dieses Düsenkörpers 46 ist eine sich kegelstumpfförmig erweiternde Abgabeöffnung 48 vorgesehen, welche - im Zusammenwirken mit einem komplementär kegelstumpfförmigen Düsen-Verschlußkörper 50 - eine normalerweise hermetisch dicht verschlossene, sich jedoch während des Applikationsvorgangs öffnende Sprühdüse bildet.

Der Hohlraum im Düsenkörper 46 hat die Form einer an die Öffnung 48 anschließenden langgestreckten, im Durchmesser gestuften zylindrischen Bohrung 52. Das behälterseitig offene Ende des Bohrungsabschnitts 52a größeren Durchmessers greift über den zylindrischen Ansatz 24 der Deckwand 22 des Behälters 12 und ist auf diesem in Behälter-Längsrichtung verschieblich geführt. Im behälterabgewandten, im lichten Durchmesser verringerten Abschnitt 52b der Bohrung 52 ist der einen Teil des Auslaßventils der Dosierpumpe 14 bildende Ventil-Zylinder 54 vorgesehen, dessen düsenseitig geschlossenes Ende von einem verschieblich durch eine Bohrung im geschlossenen Ende geführten, am Düsen-Verschlußkörper 50 angesetzten Schaft 56 durchsetzt wird. In der Wandung des Ventil-Zylinders 54 ist wenigstens eine Durchtrittsöffnung 58 zu einem auf der Außenseite zwischen dem Ventil-Zylinder 54 und dem Bohrungsabschnitt 52b gebildeten Kanal 60 vorgesehen, welcher zur Öffnung 48 der Düse führt. Die Durchtrittsöffnung(en) 58 sind normalerweise von einem Ventil-Kolben 62 verschlossen, welcher auf dem inneren Ende des Schafts 56 befestigt ist. Der Ventil-Zylinder und der mit diesem über den Schaft 56 verbundene Düsen-Verschlußkörper 50 werden normalerweise durch eine zwischen dem Ventil-Kolben 62 und dem geschlossenen Ende des Ventil-Zylinders 54 angeordnete Ventil-Feder 64 jeweils in die die Durchtrittsöffnung(en) 58 bzw. die Düsen-Öffnung 48 verschließende Stellung vorgespannt. Erst bei einem auf der düsenabgewandten Seite auf den Ventil-Kolben 62 einwirkenden Druck, welcher den Ventil-Kolben entgegen der Vorspannung der Ventil-Feder 64 verschiebt, werden die Durchtrittsöffnung(en) 58 und die Düsen-Öffnung 48 geöffnet. Damit dieser Druck beim Verschieben der Sprühdüse 16 in Abwärtsrichtung aufgebaut werden kann, ist in die Bohrung 52 ein durchbohrter Pumpenkolben 66 eingesetzt, der an seinem auslaßventilseitigen Ende am Ventil-Zylinder 54 abgestützten und an seinem gegenüberliegenden einlaßventilseitigen Ende ins hohle Innere des Behälter-Ansatzes 24 verschieblich und abgedichtet eingreift. Der Pumpenkolben 66 wird durch eine innerhalb des hohlen Ansatzes 24 abgestützte Pumpen-Feder 68 in die in der Zeichnung dargestellte Ausgangsstellung vorgespannt. Durch Herabdrücken der Sprühdüse derart, daß die Überkappe 44 weiter über die Behälterwandung 20 geschoben wird, wird der Pumpenkolben 66 ins Innere des Ansatzes 24 geschoben, wodurch in der in diesem Bereich eingeschlossenen Flüssigkeit ein Überdruck entsteht, der einerseits die Ventilkugel 32 auf die zugeordnete Sitzfläche 30 drängt und somit das Einlaßventil 26 schließt und andererseits den Ventil-Kolben 62 entgegen der Spannung der Feder 64 verschiebt. Der Ventil-Kolben 62 gibt dann nach kurzem Hub die Durchtrittsöffnung(en) 58 im Ventil-Zylinder 54 frei, und gleichzeitig wird der Düsen-Verschlußkörper 50 von der Düsen-Öffnung 48 abgehoben, so daß dann bei weiterer Pumpbetätigung die im Ansatz 24 eingeschlossene Flüssigkeit über die Durchgangsbohrung 70 im Pumpenkolben durch die Durchtrittsöffnungen 58, den Kanal 60 zur geöffneten Düse 48, 50 verdrängt wird und - bei geeigneter Ausgestaltung dieser Düse - fein verstäubt abgegeben wird. Die Menge des Sprühstrahls bei einmaliger Betätigung ergibt sich dabei aus den konstruktiver. Abmessungen des Hubs und der Abmessung des Arbeitsraums der Dosierpumpe 14.

In der Zeichnung ist innerhalb des Behälter-Ansatzes 24 noch ein Zentrier-Dorn 72 dargestellt, welcher die Pumpenfeder mittig ausgerichtet im Ansatz 24 zentriert, wobei der in Richtung zur Ventil-Kugel 32 vortretende Ansatz dieses Zentrier-Dorns den Öffnungshub der Ventil-Kugel 32 begrenzt und diese sichert.

Aus der Zeichnung ist auch erkennbar, daß der die Sprühflüssigkeit aufnehmende Raum des Behälters und der Kolben im Hinblick auf einen möglichst geringen Totraum optimiert sind. So ist im Kolben 24 eine zum kegelstumpfförmigen Vorsprung 24 komplementäre Vertiefung vorgesehen und im Randbereich der Begrenzungswand ist im Übergang zum zylindrischen Abschnitt 20 eine umlaufende ringförmige Vertiefung ausgebildet, in welche der deckwandseitige, als Lippendichtung 36 ausgebildete Rand des Kolbens 34 eintreten kann. Die im Behälter abgefüllte zu versprühende Flüssigkeit kann also bis auf einen geringen in der Pumpe verbleibenden Rest vollständig versprüht werden. Dabei ist dann auch noch festzuhalten, daß der korrekt dosierte Sprühauftrag aus der erfindungsgemäßen Applikations-Vorrichtung keine spezielle Lage des Behälters voraussetzt, d.h. daß die Vorrichtung auch in schräger oder auf dem Kopf stehender Stellung voll funktionsfähig ist.

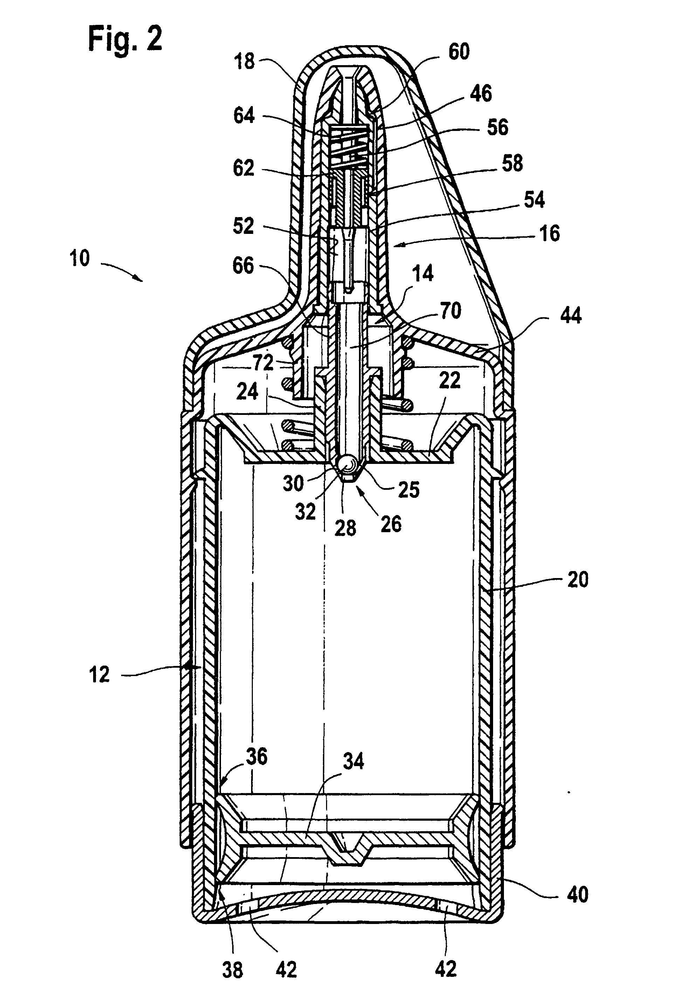

In Fig. 2 ist ein zweites Ausführungsbeispiel der erfindungsgemäßen Applikations-Vorrichtung beschrieben, welches in seinem Aufbau und der Funktion weitgehend der vorstehend in Verbindung mit Fig. 1 beschriebenen Applikations-Vorrichtung entspricht. In beiden Zeichnungsfiguren sind für funktionell gleiche Teile auch gleiche Bezugszeichen verwendet, so daß es genügt, nachstehend nur die getroffenen Abwandlungen im einzelnen zu beschreiben, während im übrigen auf die vorausgehende Beschreibung der Fig. 1 verwiesen werden kann.

Die bei der in Fig. 2 dargestellten Applikations-Vorrichtung 10 getroffenen Abwandlungen gegenüber der Applikations-Vorrichtung gemäß Fig. 1 betreffen im wesentlichen die Anordnung der den Kolben 66 der Dosierpumpe 14 federnd in eine Hub-Endstellung vorspannenden Schraubenfeder 68. Während diese Schraubenfedern beim bereits beschriebenen Ausführungsbeispiel innerhalb des Ansatzes 24 angeordnet und am Pumpenkolben 66 einerseits und oberhalb des Einlaßventils 26 im Ansatz 24 andererseits abgestützt ist und durch den Zentrierdorn 72 im Ansatz zentriert wird, ist die Schraubenfeder bei dem in Fig. 2 dargestellten Ausführungsbeispiel außerhalb des Ansatzes 24 angeordnet und wird auf der Begrenzungswand 22 einerseits und an der Innenseite der Überkappe 44 andererseits abgestützt.

Ein wesentlicher Unterschied des Ausführungsbeispiels gemäß Fig. 2 liegt darin, daß der von einer Durchgangsöffnung 70 durchsetzte Pumpenkolben 66 fest in den Ansatz 24 eingesetzt ist und an seinem behälterseitigen Ende 20 das Einlaßventil 26 trägt, welches beim Ausführungsbeispiel gemäß Fig. 1 im kegelstumpfförmigen Vorsprung 25 des Ansatzes 24 angeordnet war. Der Pumpenkolben 66 seinerseits greift am gegenüberliegenden Ende in den gleichzeitig einen Teil des Auslaßventils der Dosierpumpe 14 bildenden Zylinder 54 ein, in dem wiederum die wenigstens eine Durchtrittsöffnung 58 zu dem zwischen dem Zylinder 54 und der Innenseite des Düsenkörpers gebildeten Kanal 60 vorgesehen ist, welcher zur Öffnung 48 der Düse führt.

Der Pumpenkolben 66 wird - wie bereits erwähnt - durch die in diesem Fall an der Überkappe 44 einerseits und der Begrenzungswand 22 andererseits abgestützte Pumpen-Feder 68 in die in der Zeichnung dargestellte Ausgangsstellung vorgespannt. Durch Herabdrücken der Sprühdüse 10 derart, daß die Überkappe weiter über die Behälterwandung 20 geschoben wird, wird der Pumpenkolben 66 ins Innere des Zylinders 54 geschoben, wodurch in der in diesem Bereich eingeschlossenen Flüssigkeit ein Überdruck entsteht, der einerseits die Ventilkugel 32 auf die zugeordnete Sitzfläche 30 drängt und somit das Einlaßventil 26 schließt und andererseits den Ventil-Kolben 62 entgegen der Spannung der ihn beaufschlagenden Feder 64 verschiebt. Der Ventil-Kolben 52 gibt also nach kurzem Hub die Durchtrittsöffnung(en) 58 im Zylinder 54 frei und gleichzeitig wird der Düsen-Verschlußkörper 50 von der Düsen-Öffnung 48 abgehoben, so daß dann bei weiterer Betätigung die im Zylinder 54 und im hohlen Pumpen-Kolben 66 eingeschlossene Flüssigkeit durch die Durchtrittsöffnung(en) 58, den Kanal 60 zur geöffneten Düse 48, 50 verdrängt wird und - wiederum bei geeigneter Ausgestaltung dieser Düse - fein verstäubt abgegeben wird. In der Zeichnung ist innerhalb der Überkappe 44 noch eine zur Zentrierung der Pumpenfeder dienende, von der Innenseite der Überkappe 44 in Abwärtsrichtung vorspringende Ringwand 72 dargestellt.

Durch die Anordnung der Schraubenfeder 68 außerhalb des Ansatzes 24 entfällt der beim Ausführungsbeispiel gemäß Fig. 1 vorgesehene, die Schraubenfeder zentrierende Dorn 72. Außerdem wird durch diese Anordnung der Schraubenfeder 68 außerhalb des Zylinders der Dosierpumpe der Arbeitsraum der Dosierpumpe nicht durch das Volumen der Schraubenfeder verkleinert und auch die Durchströmung der Dosierpumpe nicht mehr durch die Schraubenfeder beeinträchtigt. Bei der Wahl des Materials der Schraubenfeder muß somit dessen Verträglichkeit mit dem zu applizierenden Fluid nicht berücksichtigt werden, weil die Feder nicht mit der zu applizierenden Flüssigkeit in Berührung kommt.

## Patentansprüche

1. Applikations-Vorrichtung (10) für keimfreie Fluide, insbesondere für pharmazeutisch wirksame Flüssigkeiten, bestehend aus einem das keimfrei abgefüllte Fluid ohne Luftraum aufnehmenden Behälter (12), in dessen abgabeseitiger Begrenzungswand (22) eine luftausgleichsfrei arbeitende, als Hubkolbenpumpe ausgebildete Dosierpumpe (14) mit einem eine Einlaßöffnung (28) zum Behälter verschließenden Einlaßventil (26) und einem abgabeseitig eine zu einer Abgabeöffnung (48) geführte Verbindung verschließenden Auslaßventil (54, 58; 62) vorliegt, wobei der Kolben der Dosierpumpe federnd in eine Hub-Endstellung vorgespannt ist, aus welcher er durch ein relativ zum Behälter verschiebliches, mit der Abgabeöffnung (48) versehenes Betätigungselement durch den Benutzer von Hand in die andere Hub-Endstellung verschieblich ist, wobei durch den bei der Hubbetätigung in dem in der Pumpe (14) eingeschlossenen Fluid entstehenden Druck das Einlaßventil (26) geschlossen und das Auslaßventil (54; 58; 62) geöffnet wird,
**dadurch gekennzeichnet,**
**daß** die den gefüllten Behälter (12) auf der der Einlaßöffnung gegenüberliegenden Seite verschließende Wand als im Behälter (12) verschieblich geführter und entlang seines Umfangs an der Innenwandung des Behälters (12) abdichtender Kolben (34) ausgebildet ist, und
**daß** der Ventilkörper (62) des Auslaßventils mit einem sich kegelstumpfförmig erweiternden Düsen-Verschlußkörper (50) verbunden ist, dessen Kegelstumpf-Umfangsfläche in der Ventil-Schließstellung an einem sich komplementär kegelstumpförmig erweiternden Bereich der Abgabeöffnung (48) abdichtend anliegt.

2. Applikations-vorrichtung nach Anspruch 1, **dadurch gekennzeichnet, daß** der Kolben (34) entlang seines behäl terinnenseitig an der Innenwandung des Behälters (12) anliegenden Randes eine umlaufende Lippendichtung (36) aufweist.

3. Applikations-Vorrichtung nach Anspruch 1 oder 2, **dadurch gekennzeichnet, daß** der Kolben (34) entlang seines behälteraußenseitig an der Innenwandung des Behälters (12 anliegenden Randes eine umlaufende Lippendichtung (38) aufweist.

4. Applikations-Vorrichtung nach Anspruch 2 oder 3, **dadurch gekennzeichnet, daß** die Lippendichtung(en) (36; 38) unter elastischer Vorspannung an die Innenwandung des Behälters (12) angedrückt ist bzw. sind.

5. Applikations-Vorrichtung nach einem der Ansprüche 1 bis 4, **dadurch gekennzeichnet, daß** der rechtwinklig zur Verschiebungsrichtung des Kolbens (34) verlaufende lichte innere Querschnitt des Behälters (12) in seinem den Kolben verschieblich aufnehmenden Bereich (20) sowie die äußere Begrenzung des Kolbens (34) komplementär geometrisch begrenzt sind.

6. Applikatons-Vorrichtung nach Anspruch 5, **dadurch gekennzeichnet, daß** der den Kolben verschieblich aufnehmende Bereich (20) des Behälters (12) und der Kolben (34) kreisförmig, elliptisch oder polygonal mit abgerundeten Polygonecken begrenzt sind.

7. Applikations-Vorrichtung nach einem der Ansprüche 1 bis 6, **dadurch gekennzeichnet, daß** an der ins Behälterinnere weisenden Kegelstumpf-Stirnfläche des mit dem Ventilkörper (62) des Auslaßventils verbundenen Düsen-Verschlußkörpers (50) ein langgestreckter Schaft angesetzt ist, der mit dem kolbenartig ausgebildeten, in einer zylindrischen Sitzfläche verschieblich geführten Ventilkörper (62) des Auslaßventils verbunden ist, und daß der Ventilkörper (62) durch eine Feder (64) in eine Endstellung vorgespannt ist, in welcher die Kegelstumpf-Mantelfläche des Düsen-Verschlußkörpers (50) am komplementär kegelstumpfförmigen Bereich der Abgabeöffnung (48) anliegt und der Ventilkörper (62) eine oder mehrere Durchlaßöffnung(en) (58) in der Wandung der Sitzfläche verschließt, wobei die Durchlaßöffnung(en) (58) über wenigstens einen anschließenden Kanal (60) mit der Abgabeöffnung (48) verbunden ist bzw. sind.

8. Applikations-Vorrichtung nach einem der Ansprüche 1 bis 7, **dadurch gekennzeichnet, daß** der Kolben (66) der Dosierpumpe (14) durch eine Schraubenfeder (68) in die Hub-Endstellung vorgespannt ist.

9. Applikations-vorrichtung nach Anspruch 8, **dadurch gekennzeichnet, daß** die Schraubenfeder (68) im Innern eines den Zylinder der Dosierpumpe (14) bildenden hohlen Ansatzes (24) der Begrenzungswand (22) angeordnet ist.

10. Applikations-Vorrichtung nach Anspruch 8, **dadurch gekennzeichnet, daß** die Schraubenfeder (68) einen von der Begrenzungswand (22) des Behälters (12) vortretenden Ansatz (24) im wesentlichen konzentrisch umgibt und auf der Außenseite der Begrenzungswand (22) einerseits und am Betätigungselement (Überkappe 44) andererseits abgestützt ist.

11. Applikations-Vorrichtung nach Anspruch 7 und 10, **dadurch gekennzeichnet, daß** der eine Durchgangsöffnung (70) aufweisende langgestreckte Kolben (66) der Dosierpumpe (14) an seinem behälterseitigen Endbereich in dem im wesentlichen hohlzylindrischen Ansatz (24) gehalten und an seinem ins Behälterinnere weisenden Ende mit dem Einlaßventil (26) versehen ist, während sein behälterabgewandtes Ende verschieblich ins Innere des in der Sprühdüse (16) angeordneten Zylinders (54) eingreift, in dessen behälterabgewandten Endbereich der Ventilkörper (62) des Auslaßventils angeordnet ist.

## Claims

1. Application device (10) for germ-free fluids, in particular for pharmaceutically effective liquids, comprising a container (12) which holds the fluid, which has been poured in in a germ-free manner, without any space for air and in the discharge-side boundary wall (22) of which there is a metering pump (14) which operates without air equalization and is designed as a reciprocating piston pump and has an inlet valve (26), which closes an inlet opening (28) to the container, and an outlet valve (54, 58; 62) which, on the discharge side, closes a connection leading to a discharge opening (48), the piston of the metering pump being prestressed resiliently into a stroke end position, from which it can be displaced into the other stroke end position by the user by hand by means of an actuating element which can be displaced relative to the container and is provided with the discharge opening (48), with the inlet valve (26) being closed and the outlet valve (54; 58; 62) being opened by the pressure arising during the stroke actuation in the fluid enclosed in the pump (14), **characterized in that** the wall closing the filled container (12) on the side opposite the inlet opening is designed as a piston (34), which is guided displaceably in the container (12) and, along its circumference, provides a seal on the inner wall of the container (12), and **in that** the valve body (62) of the outlet valve is connected to a frustoconically expanding nozzle closing body (50), the frustoconical circumferential surface of which bears in the valve-closing position in a sealing manner against a complementarily frustoconically expanding region of the discharge opening (48).

2. Application device according to Claim 1, **characterized in that** the piston (34) has an encircling lip seal (36) along its edge bearing on the inside of the container against the inner wall of the container (12).

3. Application device according to Claim 1 or 2, **characterized in that** the piston (34) has an encircling lip seal (38) along its edge bearing on the outside of the container against the inner wall of the container (12).

4. Application device according to Claim 2 or 3, **characterized in that** the lip seal(s) (36; 38) is or are pressed against the inner wall of the container (12) under elastic prestress.

5. Application device according to one of Claims 1 to 4, **characterized in that** the clear, inner cross section of the container (12) running at right angles to the displacement direction of the piston (34), in its region (20) displaceably accommodating the piston, the outer boundary of the piston (34) and have a geometrically complementary boundary.

6. Application device according to Claim 5, **characterized in that** that region (20) of the container (12) which displaceably accommodates the piston, and the piston (34) have circular, elliptical or polygonal boundaries with rounded polygon corners.

7. Application device according to one of Claims 1 to 6, **characterized in that** an elongated stem is fitted to the frustoconical end surface, which points into the interior of the container, of the nozzle-closing body (50), which is connected to the valve body (62) of the outlet valve, the said stem being connected to the valve body (62) of the outlet valve, which valve body is designed in the manner of the piston and is guided displaceably in a cylindrical seat surface, and **in that** the valve body (62) is prestressed by a spring (64) into an end position, in which the frustoconical circumferential surface of the nozzle-closing body (50) bears against the complementarily frustoconical region of the discharge opening (48), and the valve body (62) closes one or more passage openings (58) in the wall of the seat surface, the passage openings (58) being connected to the discharge opening (48) via at least one adjoining channel (60).

8. Application device according to one of Claims 1 to 7, **characterized in that** the piston (66) of the metering pump (14) is prestressed into the stroke end position by a helical spring (68).

9. Application device according to Claim 8, **characterized in that** the helical spring (68) is arranged in the interior of a hollow projection (24) of the boundary wall (22), which projection forms the cylinder of the metering pump (14).

10. Application device according to Claim 8, **characterized in that** the helical spring (68) essentially concentrically surrounds a projection (24) protruding from the boundary wall (22) of the container (12), and is supported on the outside of the boundary wall (22), on the one hand, and on the actuating element (overcap 44), on the other hand.

11. Application device according to Claims 7 and 10, **characterized in that** the elongated piston (66) of the metering pump (14), which piston has a passage opening (70), is held on its container-side end region in the essentially hollow cylindrical projection (24) and is provided with the inlet valve (26) at its end pointing into the container interior while its end facing away from the container engages displaceably in the interior of the cylinder (54) which is arranged in the spray nozzle (16) and in the end region of which, which faces away from the container, the valve body (62) of the outlet valve is arranged.

## Revendications

1. Dispositif d'application (10) pour fluides stériles, en particulier pour des liquides pharmaceutiquement actifs, formé d'un réservoir (12) contenant le fluide introduit en un état stérile, sans aucun volume d'air, réservoir dans la paroi de délimitation (22), située côté distribution, duquel est prévue une pompe de dosage (14), réalisée sous la forme de pompe à piston alternatif, travaillant sans compensation de l'air, avec une soupape d'admission (26), fermant une ouverture d'admission (28) par rapport au réservoir, et une soupape d'échappement (54, 58; 62), fermant, côté distribution, une liaison guidée à une ouverture de distribution (48), le piston de la pompe de dosage étant précontraint élastiquement vers une position finale de course, d'où, au moyen d'un élément d'actionnement mobile en translation par rapport au réservoir, muni de l'ouverture de distribution (48), il est déplaçable par l'utilisateur à la main, en translation jusqu'à l'autre position finale de course, la soupape d'admission (26) étant fermée et la soupape d'échappement (54; 58; 62) étant ouverte, au moyen de la pression, régnant lors de l'actionnement de course dans le fluide enfermé dans la pompe (14), **caractérisé en ce que** la paroi, obturant le réservoir (12) rempli, sur le côté opposé à l'ouverture d'admission, est réalisée sous forme de piston (34) guidé en translation dans le réservoir (12) et créant une étanchéité, le long de sa périphérie, sur la paroi intérieure du réservoir (12), et **en ce que** le corps de soupape (62) de la soupape d'échappement est relié à un corps de fermeture de buse (50) allant en s'élargissant en forme de tronc de cône, dont la surface périphérique en tronc de cône, à la position de fermeture de soupape, s'appuie de façon étanche sur une zone, allant en s'élargissant de façon complémentaire en forme de tronc de cône, de l'ouverture de distribution (48).

2. Dispositif d'application selon la revendication 1, **caractérisé en ce que** le piston (34) présente le long de son bord, en appui, du côté intérieur du réservoir, sur la paroi intérieure du réservoir (12), un joint d'étanchéité à lèvre (36) l'entourant.

3. Dispositif d'application selon la revendication 1 ou 2, **caractérisé en ce que** le piston (34) présente le long de son bord, en appui, côté extérieur du réservoir, sur la paroi intérieure du réservoir (12), un joint d'étanchéité à lèvre (38) l'entourant.

4. Dispositif d'application selon la revendication 2 ou 3, **caractérisé en ce que** le ou les joint(s) d'étanchéité à lèvre (36; 38) est/sont pressé(s) avec précontrainte élastique sur la paroi intérieure du réservoir (12).

5. Dispositif d'application selon l'une des revendications 1 à 4, **caractérisé en ce que** la section transversale intérieure libre, s'étendant à angle droit de la direction de translation du piston (34), du réservoir (12), dans sa zone (20) recevant le piston de façon mobile en translation, ainsi que la délimitation extérieure du piston (34) sont délimitées de façon géométriquement complémentaire.

6. Dispositif d'application selon la revendication 5, **caractérisé en ce que** la zone (20), recevant le piston de façon mobile en translation, du réservoir (12) et le piston (34) sont délimités, en forme de cercle, de façon elliptique ou polygonale, avec des angles de polygone arrondis.

7. Dispositif d'application selon l'une des revendications 1 à 6, **caractérisé en ce que**, sur la face frontale de tronc de cône, tournée vers l'intérieur du réservoir, du corps de fermeture de buse (50), relié au corps de soupape (62) de la soupape d'échappement, est appliquée une tige allongée, qui est reliée au corps de soupape (62) de la soupape d'échappement, configuré en piston, guidé de façon mobile dans une face de portée cylindrique et **en ce que** le corps de soupape (62) est précontraint par un ressort (64) vers une position finale, à laquelle la face d'enveloppe tronconique du corps de fermeture de buse (50) vient en appui, sur la zone, à forme tronconique complémentaire, de l'ouverture de distribution (48), et le corps de soupape (62) ferme une ou plusieurs ouvertures (58) ménagées dans la paroi de la face de portée, la ou les ouvertures (58) étant reliée(s) à l'ouverture de distribution (48) par au moins un canal (60) de raccordement.

8. Dispositif d'application selon l'une des revendications 1 à 7, **caractérisé en ce que** le piston (66) de la pompe de dosage (14) est précontraint vers la position finale de course au moyen d'un ressort hélicoïdal (68).

9. Dispositif d'application selon la revendication 8, **caractérisé en ce que** le ressort hélicoïdal (68) est disposé à l'intérieur d'un appendice (24) creux, formant le cylindre de la pompe de dosage (14), de la paroi de délimitation (22).

10. Dispositif d'application selon la revendication 8, **caractérisé en ce que** le ressort hélicoïdal (68) entoure, de façon sensiblement concentrique, un appendice (24) placé à l'avant de la paroi de délimitation (22) du réservoir (12) et est appuyé, d'une part, sur la face extérieure de la paroi de délimitation (22) et, d'autre part, sur l'élément d'actionnement (capuchon d'habillage 44).

11. Dispositif d'application selon les revendications 7 et 10, **caractérisé en ce que** le piston (66) allongé, présentant une ouverture de passage (70), de la pompe de dosage (14) est maintenu, sur sa zone d'extrémité côté réservoir, dans l'appendice (24) sensiblement creux et cylindrique, et est muni, à son extrémité, tournée vers l'intérieur du réservoir, de la soupape d'admission (26), tandis que son extrémité opposée au réservoir s'engage de façon mobile en translation à l'intérieur du cylindre (54) disposé dans la buse de pulvérisation (16), cylindre dans la zone d'extrémité, opposée au réservoir, duquel est disposé le corps de soupape (62) de la soupape d'échappement.
